# EUROPEAN PATENT SPECIFICATION

(11) **EP 0 877 742 B1**
(45) Date of publication and mention of the grant of the patent: **16.08.2001**
(21) Application number: 96934242.7
(22) Date of filing: 22.10.1996
(51) Int. Cl.: C07D 309/30, C12P 17/06

(54) **METHOD OF PRODUCTION OF LOVASTATIN**
VERFAHREN ZUR HERSTELLUNG VON LOVASTATIN
PROCEDE DE PRODUCTION DE LOVASTATINE

(30) Priority: 06.12.1995 BG 10019795; 29.01.1996 BG 10031696
(43) Date of publication of application: 18.11.1998
(73) Proprietor: Balkanpharma-Razgrad AD, 7200 Razgrad (BG)
(72) Inventor: DIMOV, Dimcho Ivanov, 7200 Razgrad (BG); GROZDANOV, Georgy Asenov, 7200 Razgrad (BG); PETKOV, Nedelcho Genov, 7200 Razgrad (BG); TODOROVA, Dimitra Tsoneva, 7200 Razgrad (BG); DIMITROVA, Albena Stefanova, 7200 Razgrad (BG)
(74) Representative: von Füner, Alexander, Prof.h.c. Dr.
(86) International application number: BG9600013
(87) International publication number: WO9720834

(56) References cited:
- EP-A- 0 033 538
- WO-A-94/10328
- WO-A-94/29292

## Description

### Field of technics

The invention is related to a method of production of lovastatin finding application in the pharmaceutical industry.

### Preceding state of technics

Methods of isolation of lovastatin are known where the filtered culture broth, containing lovastatin, is extracted using ethyl acetate, the extract obtained is lactonized by boiling in toluene, and the isolated product is purified by chromatographing on silica gel (US 4444784, US 4450171).

A method of lovastatin isolation is known, where a sample of culture broth, mycelium or filtrate is extracted by butyl acetate, at pH 3-5, the resulting extract is concentrated through simultaneous lactonization, under vacuum, then through cooling lovastatin in lactone form is directly crystallized from it. (WO 94/29292).

A method of lovastatin isolation and purification is known, where the culture broth is alkalized to pH 8.5-9 and it is filtrated. The separated mycelium is extracted by alkaline water, at continuous agitation, it is filtrated, combined with the filtrate, obtained during culture broth filtration and it is purified through butyl acetate extraction at pH 4 or through ultrafiltration. The obtained filtrate is concentrated at simultaneous lactonization under vacuum. Gaseous ammonia is passed through the concentrate, the resulting precipitate is filtrated, and the filtrate is reconcentrated to 1/10 of its initial volume, then lovastatin in lactone from is crystallized through cooling to -20°C. The obtained crystals are filtrated, dried under vacuum at about 40°C and recrystallized in ethyl acetate, butyl acetate or ethanol. (BG 60460A)

A disadvantage of these methods is the necessity for using large volumes of extracting agents which leads to burdening expenses including also the ones for their reclamation with the view of protecting the environmental elements. The purification by extraction or by ultrafiltration/reverse osmosis demands usage of sophisticated and expensive equipment, the process are multistage, which contributes to the decrease of the yield. As a result of oxidation by-products are formed in the process of isolation of lovastatin and during the storage of the finished product obtained by any of the known methods. This leads to a decrease of the purity and the stability of the product.

Another method of lovastatin isolation and purification is known, where culture broth is diluted with water, treated at continuous agitation for 2 hours at pH 7.5-10 in presence of organic ingredient in quantity not less than 0.1 weight %, then it is filtrated. Form this filtrate at pH 1.0-4.5, in presence of salts of alkali-earth or earth metals, lovastatin is directly precipitated, it is filtrated and purified, preferably by recrystallization. (WO 94/10328).

A disadvantage of this method is the possibility of oxidation of the active ingredient during the process, as well as the high residual activity of lovastatin in mother liquor at precipitation, which is carried out in a volume considerably higher than the initial culture broth volume.

The goal of the invention is creation of a method for production of lovastatin of high purity and stability, which is simplified, reliable, and employs small quantities of solvents.

### Technical nature of the invention

The goal of the invention is achieved by a method, in which the culture broth containing lovastatin is treated with an alkaline base in the presence of an antioxidant, pH 9.5÷13.0, the mycelium is filtered and washed with a diluted solution of an alkaline base. The solution obtained is acidified with a mineral acid to pH 2.5÷4.0 in the presence of an antioxidant, inert filler and a 0.1÷0.3 % non-miscible with water organic solvent. The precipitate produced is filtered, dehydrated, extracted and lactonized by boiling in the medium of a chlorine-containing organic solvent. The latter is distilled to a high concentration of the residue and dissolved in a mixture of acetonitrile-tert.-butylmethylether-butyl chloride in the presence of an antioxidant, and after cooling to -10÷-30°C is crystallized lovastatin, which is isolated and dried.

The filtered and dried raw lovastatin is dissolved in a nitrile such as acetonitrile or propionitrile. The solution obtained is decoloured using a mixture of activated carbon and aluminium oxide. After addition of water, lovastatin is precipitated at room temperature from the filtrate obtained. The filtrate is separated and the lovastatin obtained is recrystallized twice, successively in a mixture of acetate of a lows alcohol-alkane C₇-C₉ at a temperature of 0°÷-20°C and then in acetone at a temperature of -10÷-30°C. The crystalline product obtained is filtered and dried at a temperature of 50°C, and at an underpressure of 1 kPa.

As antioxidants are used : butylhydroxtoluene, butylhydroxyanisole, nordihydroguaiaretic acid, propyl ester of gallic acid, 2,2-methylene-bis(6-tert.-butyl-4methyl-phenol), sodium pyrosulphite, sodium bisulphite, d,1-α-tocopherylacetate, hydroquinone, etc.

Silica gel, activated carbon, diatomite, kieselguhr, perlite, sawdust, wood shavings, zeolite are used as fillers for the precipitation of lovastatin.

Esters of aliphatic acids (amyl acetate, ethyl propionate, butyl formiate, butyl acetate, etc.), chlorine-containing carbon hydrides (methylene chloride, chloroform, trichloroethylene, trichloroethane, perchloroethylene, dichloroethane, etc.) are used as non-miscible with water organic solvent.

Methyl acetate, ethyl acetate, propyl acetate or butyl acetate can be used as acetates of low alcohols, as the system of propyl acetate-isooctane is recommended.

The crystallization of lovastatin is performed in a mixture of solvents of different polarity in a suitable ratio, as mixtures of isopropanol-acetone, acetonitrile-butyl chloride, acetonitrile-tert.-butylmethylether-butyl chloride are used.

Advantages of the method according to the invention are production of lovastatin of high purity and yield, stable during storage, by using a simplified technological scheme and small quantities of solvents.

### Examples for performance of the invention

To culture broth, containing 792 g of lovastatin (calculated as lactone), are added 16.3 g of butylhydroxytoluene in 165 ml of ethyl alcohol, the mixture is alkalified with a 40 % sodium hydroxide to pH 11.5 and stirred for 3 hours. The mycelium is filtered, washed six-fold - each time with 100 1 of a 0.05 % sodium hydroxide. To the solution obtained are added 20 g of butylhydroxytoluene and 10 kg of perlite, and the mixture is acidified with a 20 % nitric acid to pH 2.7. After stirring for 30 min, 18 1 of chloroform are added, and the mixture is stirred for another 1 h. The residue is separated from the filter-press. The product obtained is suspended in 100 1 of chloroform and boiled in order to be to dehydrated using a metal Florence flask type of vessel until lovastatin acid is lactonized thoroughly. The process is monitored by HPLC. The chloroform solution is distilled to the consistence of oil which is dissolved in a mixture of 0.5 1 of acetonitrile, 0.5 1 of tert.-butylmethylether and 3.0 1 of butyl chloride. After cooling to -20°C for 48 h the product crystallizes.

After filtration and drying, the raw lovastatin is dissolved in 10 1 of propionitrile at 35°C, decoloured using 280 g of activated carbon and 280 g aluminium oxide, and after filtration it is precipitated with 46 1 of water at room temperature for 2 h. The filtered product is dissolved at 70°C in 3 1 of propyl acetate, and after addition of 4 1 of isooctane it is cooled to -20°C for 30 h. The filtered product is recrystallized in 2.5 1 of acetone at a final temperature of -30°C, filtered and dried at a temperature of 50 °C and at an underpressure of 1 kPa.
601 g of lovastatin of 99.5 % purity are produced (HPLC).
Yield of lovastatin from culture broth - 75.5 %.
Optical rotation - -330° (c=5.0 mg/ml, CH₃CN).
IR and NMR spectra of the product are identical to these of Lovastatin USP Reference Standard.

## Claims

1. Method of production of lovastatin characterized by that, that lovastatin is derived from culture broth at a pH value of 9.5÷13.0, the mycelium is filtered, washed with a diluted alkaline base, and included in a solid mass lovastatin is precipitated in the filtrate,pH 2.5÷4.0, in the presence of an inert filler, antioxidant and a 0.1÷3.0 % non-miscible with water organic solvent, the precipitate is filtered, dehydrated azeotropically and lactonized by boiling in a medium of a chlorine-containing organic solvent, then the solvent is distilled to a high concentration of the residue, dissolved in a mixture of solvents of different polarity, the solution produced is cooled to -10÷-30°C, and the crystallized lovastatin is isolated, dried and recrystallized successively in a mixture of nitrile-water, by decolouring with aluminium oxide and activated carbon, and in a mixture of acetate of a low alcohol-alcane C₇-C₉ at a temperature of 0÷-20°C and in acetone at -10÷-30°C.

2. Method according to **Claim 1**, characterized by that, that butylhydroxytoluene, butylhydroxyanisole, nordihydroguaiaretic acid, propyl ester of gallic acid, 2,2-methylene-bis-(6-tert.-butyl-4-methyl-phenol), sodium pyrosulphite, sodium bisulphite, d,1-α-tocopheryl acetate, hydroquinone can be used as antioxidants.

3. Method according to **Claim 1** characterized by that, that methylene chloride, chloroform, trichloroethylene, trichloroethane, perchloroethylene, dichloroethane can be used as a chlorine-containing solvent for extraction and lactonization.

4. Method according to **Claim 1** and **Claim 2** characterized by that, that the following mixtures of solvents : Isopropanol-acetone,Acetonitrile-butyl chloride,Acetonitrile-tert.-butylmethylether-butyl chloride, Propyl acetate-isooctane, Ethyl acetate-n-heptane, can be used for dissolution and crystallization.

## Patentansprüche

1. Verfahren zur Herstellung von Lovastatin, dadurch **gekennzeichnet**, daß Lovastatin aus einer Kulturbouillon bei einem pH-Wert von 9,5 bis 13,0 gewonnen wird, das Mycel abfiltriert und mit verdünnter Alkalilauge gewaschen wird, das in einer Feststoffmasse eingeschlossene Lovastatin im Filtrat bei einem pH von 2,5 bis 4,0 in Anwesenheit eines inerten Füllers, eines Antioxydans und eines 0,1 bis 3,0%igen mit Wasser nicht mischbaren organischen Lösungsmittels ausgefällt wird, der Niederschlag abfiltriert, azeotrop dehydratisiert und durch Kochen in einem Medium aus einem chlorhaltigen organischen Lösungsmittel laktonisiert wird, das Lösungsmittel anschließend bis zu einer starken Einengung des Rückstandes abdestilliert wird, wonach in einem Lösungsmittelgemisch von unterschiedlicher Polarität gelöst wird, die erhaltene Lösung auf -10 bis -30°C abgekühlt wird, dann das kristallisierte Lovastatin isoliert, getrocknet und anschließend in einem Gemisch aus Nitril und Wasser unter Entfärbung mit Aluminiumoxid und Aktivkohle und dann in einem Gemisch aus einem Acetat eines C₇₋₉-Niederalkanols bei einer Temperatur von 0 bis -20°C und in Aceton bei -10 bis -30°C umkristallisiert wird.

2. Verfahren nach Anspruch 1, dadurch **gekennzeichnet**, daß als Antioxydantien Butylhydroxytoluol, Butylhydroxyanisol, Nordihydroguajaretsäure, Gallsäurepropylester, 2,2-Methylen-bis-(6-tert.-butyl-4-methyl-phenol), Natriumpyrosulfit, Natriumbisulfit, d,1-α-Tocopherylacetat und Hydrochinon verwendet werden können.

3. Verfahren nach Anspruch 1, dadurch **gekennzeichnet**, daß als chlorhaltiges Lösungsmittel für die Extraktion und die Laktonisierung Methylenchlorid, Chloroform, Trichlorethylen, Trichlorethan, Perchlorethylen und Dichlorethan verwendet werden können.

4. Verfahren nach Anspruch 1 und Anspruch 2, dadurch **gekennzeichnet**, daß zur Lösung und Kristallisation folgende Lösungsmittelgemische verwendet werden können: Isopropanol-Aceton, Acetonitril-Butylchlorid, Acetonitriltert.-Butylmethylether-Butylchlorid, Propylacetat-Isooctan und Ethylacetat-n-Heptan.

## Revendications

1. Procédé de préparation de lovastatine, caractérisé en ce qu'on dérive de la lovastatine d'un bouillon de culture à une valeur de pH de 9,5 à 13,0, on filtre le mycélium, on lave avec une base alcaline diluée, et on précipite la lovastatine incluse dans la matière solide dans le filtrat à un pH de 2,5 à 4,0 en présence d'une matière de charge inerte, d'un antioxydant et d'un solvant organique non miscible à l'eau à concurrence de 0,1 à 3,0 %, on filtre le précipité, on le soumet à une déshydratation par voie azéotrope et à une lactonisation par ébullition dans un solvant organique chloré, puis on sépare le solvant par distillation pour obtenir une concentration élevée du résidu, on dissout dans un mélange de solvants possédant des polarités différentes, on refroidit à une température de -10 à -30°C la solution obtenue et on isole la lovastatine cristallisée, on sèche et on recristallise successivement dans un mélange nitrile-eau, en soumettant à une décoloration avec de l'oxyde d'aluminium et du charbon activé, dans un mélange d'acétate et d'un alcanol inférieur en C₇-C₉ à une température de 0 à -20°C et dans de l'acétone à une température de -10 à -30°C.

2. Procédé selon la revendication 1, caractérisé en ce qu'on peut utiliser, à titre d'antioxydants, le butylhydroxytoluène, le butylhydroxyanisole, l'acide nordihydroguaiarétique, l'ester propylique de l'acide gallique, le 2,2-méthylène-bis-(6-tert.-butyl-4-méthyl-phénol), le pyrosulfite de sodium, le bisulfite de sodium, le d,l-α-tocophéryl-acétate, l'hydroquinone.

3. Procédé selon la revendication 1, caractérisé en ce qu'on peut utiliser, à titre de solvant chloré pour l'extraction et la lactonisation, le chlorure de méthylène, le chloroforme, le trichloréthylène, le trichloréthane, le perchloréthylène, le dichloréthane.

4. Procédé selon les revendications 1 et 2, caractérisé en ce qu'on peut utiliser pour la dissolution et pour la cristallisation, les mélanges de solvants ci-après: des mélanges d'isopropanol - acétone, d'acétonitrile - chlorure de butyle, d'acétonitrile - éther tert.-butylméthylique- chlorure de butyle, d'acétate de propyle - isooctane, d'acétate d'éthyle - n-heptane.
